(19) Europäisches Patentamt
European Patent Office
Office européen des brevets

(11) EP 3 660 486 A1

(12) **EUROPEAN PATENT APPLICATION**
published in accordance with Art. 153(4) EPC

(43) Date of publication:
**03.06.2020 Bulletin 2020/23**

(21) Application number: **18837825.1**

(22) Date of filing: **25.07.2018**

(51) Int Cl.:
***G01N 5/02*** *(2006.01)* ***G01N 27/22*** *(2006.01)*

(86) International application number:
**PCT/JP2018/027866**

(87) International publication number:
**WO 2019/022124 (31.01.2019 Gazette 2019/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.07.2017 JP 2017146407**

(71) Applicant: **FUJIFILM Corporation**
**Tokyo 106-8620 (JP)**

(72) Inventors:
- **HIRONAKA Koji**
  **Ashigara-kami-gun**
  **Kanagawa 258-8577 (JP)**
- **MOCHIZUKI Fumihiko**
  **Ashigara-kami-gun**
  **Kanagawa 258-8577 (JP)**
- **TAKAKU Koji**
  **Ashigara-kami-gun**
  **Kanagawa 258-8577 (JP)**

(74) Representative: **Hoffmann Eitle**
**Patent- und Rechtsanwälte PartmbB**
**Arabellastraße 30**
**81925 München (DE)**

(54) **SENSOR**

(57) The present invention provides a sensor having excellent sensitivity and selectivity with respect to a ketone-based compound. This sensor according to the present invention has a receiving layer including a polymer having a repeating unit represented by Formula (1), and detects a ketone-based compound.

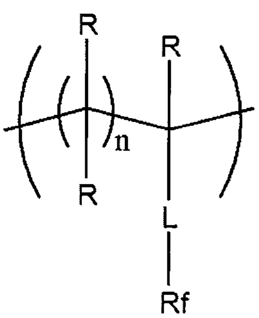

(1)

EP 3 660 486 A1

## Description

## BACKGROUND OF THE INVENTION

1. Field of the Invention

The present invention relates to a sensor.

2. Description of the Related Art

There is an increasing demand for highly sensitive detection and monitoring of rare gases floating in the air. For example, it is known that exhalation and skin gas contain a ketone-based compound and it is considered to use the result of detecting the ketone-based compound with a sensor as an index for diagnosing a health condition.

In recent years, investigations have been conducted on the use of a polymer having fluorine atoms in sensors.

For example, JP2015-007618A discloses a capacitance type gas sensor having a sensitive film formed of fluorinated polyimide.

## SUMMARY OF THE INVENTION

When the present inventors investigated whether or not the technology disclosed in JP2015-007618A could be applied to the detection of a ketone-based compound, the sensitivity and selectivity of detection considered to be effective as an index for diagnosis of the condition of health, metabolism, and the like were not obtained. That is, it was difficult to apply the technology to a low concentration and multicomponent system such as exhalation and skin gas.

Accordingly, an object of the present invention is to provide a sensor having excellent sensitivity and selectivity with respect to a ketone-based compound.

As a result of intensive investigations, the present inventors have found that the above object can be achieved by using a polymer that satisfies a predetermined requirement and has a repeating unit having a fluorine atom.

That is, the above object can be achieved by the following configuration.

[1] A sensor that detects a ketone-based compound, comprising: a receiving layer that contains a polymer having a repeating unit represented by Formula (1) shown later, in which a fluorine content of the repeating unit represented by Formula (1) shown later is 30% to 60% by mass.

[2] The sensor according to [1], in which a content of the repeating unit represented by Formula (1) shown later is 10 mol% or more with respect to all the repeating units of the polymer.

[3] The sensor according to [1] or [2], in which the fluorine content of the repeating unit represented by Formula (1) shown later is 40% to 60% by mass.

[4] The sensor according to any one of [1] to [3], in which, in Formula (1) shown later, Rf represents a hydrocarbon group having three or more fluorine atoms.

[5] The sensor according to any one of [1] to [4], in which, in Formula (1) shown later, Rf represents a fluorohydrocarbon group that has three or more fluorine atoms and is selected from the group consisting of an aryl group, an alkyl group, and a group formed by combining a plurality thereof.

[6] The sensor according to any one of [1] to [5], in which, in Formula (1) shown later, Rf represents a fluorinated aryl group having three or more fluorine atoms, a fluorinated alkyl group having three or more fluorine atoms, a fluorinated biphenyl group having three or more fluorine atoms, a fluorinated alkyl-substituted aryl group having three or more fluorine atoms, or a fluorinated benzyl group having three or more fluorine atoms.

[7] The sensor according to any one of [1] to [6], in which, in Formula (1) shown later, Rf represents a fluorinated aryl group having three or more fluorine atoms.

[8] The sensor according to any one of [1] to [7], in which, in Formula (1) shown later, Rf represents a pentafluorophenyl group.

[9] The sensor according to any one of [1] to [8], in which, in Formula (1) shown later, L represents a single bond, an ester group, an amide group, or a group that is linked to any one of R's to form a ring.

[10] The sensor according to any one of [1] to [9], in which, in Formula (1) shown later, L represents a single bond, an ester group, or an amide group.

[11] The sensor according to any one of [1] to [10] that detects a ketone-based compound contained in exhalation or skin gas.

[12] The sensor according to any one of [1] to [11], in which the ketone-based compound is acetone.

[13] The sensor according to any one of [1] to [12], further comprising: another receiving layer, in addition to the receiving layer.

[14] The sensor according to any one of [1] to [13] that is a resonant sensor.

According to the present invention, it is possible to provide a sensor having excellent sensitivity and selectivity with respect to a ketone-based compound.

**BRIEF DESCRIPTION OF THE DRAWINGS**

Fig. 1 is a cross-sectional view schematically showing an example of a resonant sensor as an embodiment of a sensor according to the present invention.
Fig. 2 is a cross-sectional view schematically showing an example of a field effect transistor type sensor as an embodiment of the sensor according to the present invention.
Fig. 3 is a view schematically showing an example of a skin gas measurement sensor as an embodiment of the sensor according to the present invention.

**DESCRIPTION OF THE PREFERRED EMBODIMENTS**

Hereinafter, a sensor according to an embodiment of the present invention will be described in detail.
The constituent requirements described below may be embodied based on the representative embodiments of the present invention. However, the present invention is not limited to such embodiments.
In the present specification, "to" denotes a range including numerical values described before and after "to" as a lower limit value and an upper limit value.
In the present specification, the weight-average molecular weight is defined as a value measured by gel permeation chromatography (GPC) and calculated in terms of polystyrene.
For example, GPC measurement is performed by using HLC-8121GPC (manufactured by Tosoh Corporation), using two items of TSKgel $GMH_{HR}$-H (20) HT (manufactured by Tosoh Corporation, 7.8 mm ID × 30 cm) as columns, and using 1,2,4-trichlorobenzene as an eluent. In addition, GPC measurement is performed by using an infrared (IR) detector under the conditions in which the sample concentration is 0.02% by mass, the flow rate is 1.0 ml/min, the sample injection amount is 300 μl, and the measurement temperature is 160°C.
A sensor according to an embodiment of the present invention is a sensor that detects a ketone-based compound and has a receiving layer containing a polymer having a repeating unit represented by Formula (1) shown below.
Hereinafter, the polymer having a repeating unit represented by Formula (1) is also referred to as "specific polymer".
A feature of the sensor according to the embodiment of the present invention is having a receiving layer containing a specific polymer. In addition, another feature is that the fluorine content (fluorine atom content) of the repeating unit represented by Formula (1) in the specific polymer is 30% to 60% by mass.
A predetermined compound is adsorbed onto the receiving layer through a certain interaction between the receiving layer and the predetermined compound.
A polymer such as polytetrafluoroethylene (PTFE) having a repeating unit having a high fluorine content (more than 60% by mass) exhibits low affinity with many organic substances and does not adsorb a ketone-based compound. On the other hand, a polymer having a repeating unit having a low fluorine content (less than 30% by mass) can exhibit affinity with many organic substances but cannot selectively detect a ketone-based compound.
When the polymer having a repeating unit represented by Formula (1) having an appropriate fluorine content (30% to 60% by mass) was tested, it was found that the specific polymer exhibited high sensitivity and high selective adsorptivity with respect to a ketone-based compound. The specific polymer having such properties is preferable as a material constituting the receiving layer of the sensor for detecting a ketone-based compound.
In a case where the specific polymer is used, although the mechanism by which the problem of the present invention is solved is not necessarily clear, the present inventors presume that the C-F bond in the repeating unit and the C=O bond of the ketone-based compound have a certain interaction with each other.

[Specific Polymer]

Hereinafter, the specific polymer will be described.

<Repeating Unit Represented by Formula (1)>

The specific polymer has a repeating unit represented by Formula (1).

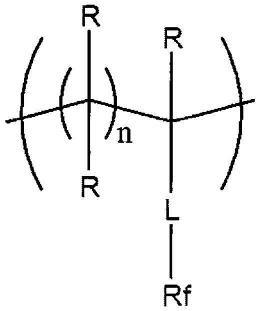

(1)

n represents an integer of 1 to 3 and is preferably 1 or 2 and more preferably 1.

R's each independently represent a hydrogen atom or an organic group and is preferably a hydrogen atom or a methyl group from the viewpoint of further improving the sensitivity and selectivity of the sensor.

In addition, R's may be linked to each other to form a ring.

L represents a single bond or a divalent linking group.

Examples of the divalent linking group include an ether group (-O-), a carbonyl group (-CO-), an ester group (-COO-), an amide group (-CONH-), a thioether group (-S-), $-SO_2-$, $-NR^F-$ ($R^F$ represents a hydrogen atom or an alkyl group), a divalent hydrocarbon group (for example, an alkylene group, an alkenylene group (for example: -CH=CH-), an alkynylene group (for example: -C≡C-), and an arylene group), and a group formed by combining these groups.

In addition, L and any one of R's may be linked to each other to form a ring. As R to be linked to L, R included in $(-C(R)(R)-)_n$ in Formula (1) is preferable. As the ring formed by linking L and any one of R's, a 5- or 6-membered non-aromatic hetero ring is preferable and a succinimide ring or a 1,3-dioxane ring is more preferable.

From the viewpoint of further improving the sensitivity and selectivity of the sensor, L preferably represents a single bond, an ester group, an amide group, or a group formed by linking L and any one of R's to form a ring, and more preferably represents a single bond, an ester group, or an amide group.

However, neither R nor L has a fluorine atom.

Rf represents an organic group having three or more fluorine atoms.

The number of fluorine atoms contained in the organic group is three or more, preferably 3 to 15, and more preferably 5 to 10 from the viewpoint of further improving the sensitivity and selectivity of the sensor.

The kind of organic group is not particularly limited and the organic group may have a predetermined number of fluorine atoms. For example, a fluorohydrocarbon group having three or more fluorine atoms may be exemplified. The fluorohydrocarbon group is a hydrocarbon group in which at least one or more hydrogen atoms are substituted with a fluorine atom.

Examples of the fluorohydrocarbon group include a fluorohydrocarbon that has three or more fluorine atoms and is selected from the group consisting of an aryl group, an alkyl group, and a group formed by combining a plurality thereof.

Examples of the fluorohydrocarbon group that has three or more fluorine atoms and is selected from the group consisting of an aryl group, an alkyl group, and a group formed by combining a plurality thereof include

an aryl group in which at least three or more hydrogen atoms are substituted with fluorine atoms, an alkyl group in which at least three or more hydrogen atoms are substituted with fluorine atoms, an aryl group having an aryl group as a substituent in which at least three or more hydrogen atoms are substituted with fluorine atoms, an aryl group having an alkyl group as a substituent in which at least three or more hydrogen atoms are substituted with fluorine atoms, and an alkyl group having an aryl group as a substituent in which at least three or more hydrogen atoms are substituted with fluorine atoms.

In the aryl group having an aryl group as the substituent, the number of aryl groups as substituents may be one or more, and may be two or more. In the aryl group having an alkyl group as the substituent, the number of alkyl groups as substituents may be one or more or two or more. In the alkyl group having an aryl group as the substituents, the number of aryl groups as substituents may be one or more or two or more.

From the viewpoint of further improving the sensitivity and selectivity of the sensor, Rf preferably represents a fluorinated aryl group having three or more fluorine atoms, a fluorinated alkyl group having three or more fluorine atoms, a fluorinated biphenyl group having three or more fluorine atoms, a fluorinated alkyl-substituted aryl group having three or more fluorine atoms, or a fluorinated benzyl group having three or more fluorine atoms.

Among these, a fluorinated aryl group having three or more fluorine atoms is preferably and a pentafluorophenyl group is more preferable.

The fluorinated aryl group is an aryl group in which at least one or more hydrogen atoms are substituted with fluorine atoms. The fluorinated alkyl group is an alkyl group in which at least one or more hydrogen atoms are substituted with fluorine atoms. The fluorinated biphenyl group is a biphenyl group in which at least one or more hydrogen atoms

are substituted with fluorine atoms. The fluorinated alkyl-substituted aryl group is an alkyl-substituted aryl group in which at least one or more hydrogen atoms are substituted with fluorine atoms. The fluorinated benzyl group is a benzyl group in which at least one or more hydrogen atoms are substituted fluorine atoms.

The alkyl-substituted aryl group means an aryl group with a substituted alkyl group, and the number of substitutions in the alkyl group may be one or more or two or more.

The number of fluorine atoms contained in the fluorinated aryl group is three or more, preferably 3 to 15, more preferably 5 to 10, and still more preferably 5 from the viewpoint of further improving the sensitivity and selectivity of the sensor.

The number of carbon (the number of carbon atoms) in the fluorinated aryl group is preferably 6 to 24, more preferably 6 to 18, and still more preferably 6 to 12.

That is, the fluorinated aryl group is preferably a pentafluorophenyl group.

The number of fluorine atoms contained in the fluorinated alkyl group is three or more and preferably 3 to 15 and more preferably 5 to 10 from the viewpoint of further improving the sensitivity and selectivity of the sensor.

The fluorinated alkyl group may be linear or branched and may have a cyclic structure.

The number of carbon (the number of carbon atoms) in the fluorinated alkyl group is preferably 1 to 10, more preferably 2 to 8, and still more preferably 3 to 6.

The number of fluorine atoms contained in the fluorinated biphenyl group is three or more, preferably 3 to 9, more preferably 5 to 9, and still more preferably 9 from the viewpoint of further improving the sensitivity and selectivity of the sensor.

That is, the fluorinated biphenyl group is preferably a nonafluorobiphenyl group.

The number of fluorine atoms contained in the fluorinated alkyl-substituted aryl group is three or more, preferably 3 to 15 and more preferably 5 to 10 from the viewpoint of further improving the sensitivity and selectivity of the sensor.

The number of carbon (the number of carbon atoms) in the fluorinated alkyl-substituted aryl group is preferably 7 to 25, more preferably 7 to 19, and still more preferably 8 to 13.

The number of fluorine atoms contained in the fluorinated benzyl group is 3 or more, preferably 3 to 15, and more preferably 5 to 10 from the viewpoint of further improving the sensitivity and selectivity of the sensor.

The substituent of the organic group is not limited to the fluorine atom and the organic group may have substituents other than the fluorine atom. For example, the organic group may have a hydroxyl group as a substituent.

In addition, the above-mentioned fluorinated aryl group having three or more fluorine atoms, fluorinated alkyl group having three or more fluorine atoms, fluorinated biphenyl group having three or more fluorine atoms, fluorinated alkyl-substituted aryl group having 3 or more fluorine atoms, and fluorinated benzyl group having three or more fluorine atoms may also have substituents other than the fluorine atom (for example, hydroxyl group).

The fluorine content of the repeating unit represented by Formula (1) is 30% to 60% by mass. The fluorine content is preferably 35% to 60% by mass and more preferably 40% to 60% by mass from the viewpoint of further improving the sensitivity and selectivity of the sensor.

The fluorine content of the repeating unit represented by Formula (1) is a ratio of the total mass of the fluorine atom having the repeating unit represented by Formula (1) with respect to the total mass of the repeating unit represented by Formula (1). Specifically, the fluorine content of the repeating unit represented by Formula (1) is obtained by the following expression.

Fluorine content (% by mass) = 100 $\times$ (total mass of fluorine atom of repeating unit represented by Formula (1)/total mass of repeating unit represented by Formula (1))

The total mass of the repeating unit represented by Formula (1) means the total mass of all atoms constituting the repeating unit represented by Formula (1).

<Repeating Unit Represented by Formula (2)>

The repeating unit represented by Formula (1) is preferably a repeating unit represented by Formula (2) from the viewpoint of further improving the sensitivity and selectivity of the sensor.

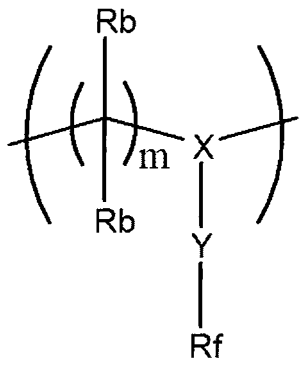

(2)

X represents a group represented by any one of Formulae (a) to (c) below.

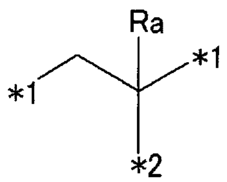

(a)

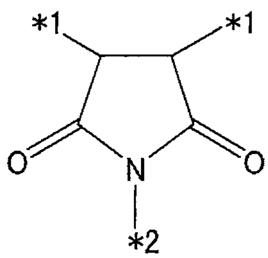

(b)

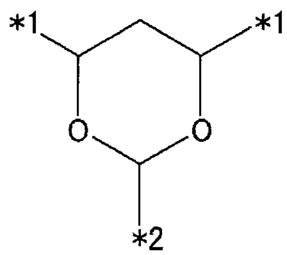

(c)

In Formula (a), Ra represents a hydrogen atom or a methyl group.
In two *1's in each of Formulae (a) to (c) represent, one represents a bonding position with $(-C(Rb)(Rb)-)_m$ and the other represents a bonding position with other repeating units.
*2 represents a bonding position with Y.
Rb's each independently represent a hydrogen atom or a methyl group.
m represents an integer of 0 to 2. However, in a case where X represents a group represented by Formula (c), m represents an integer of 0 or 1.
Y represents a single bond, an ester group, or an amide group.
Rf has the same meaning as Rf in Formula (1) and the preferable range thereof is also the same.
In addition, the fluorine content of the repeating unit represented by Formula (2) is 30% to 60% by mass, and the preferable range thereof is the same as the preferable range of the fluorine content of the repeating unit represented by Formula (1).
Examples of the repeating unit represented by Formula (1) are shown below.
The carbon atom with "·" in the repeating unit means a carbon atom bonded to an adjacent repeating unit.

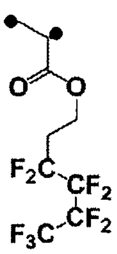
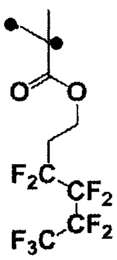
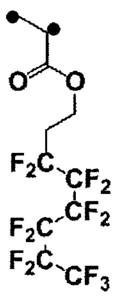
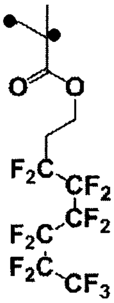

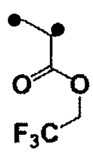
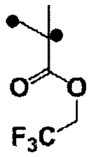
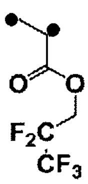
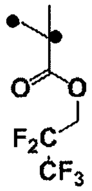

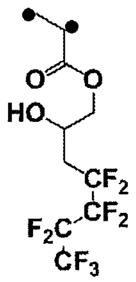 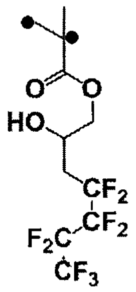 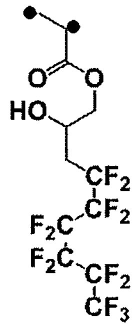 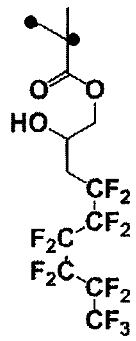

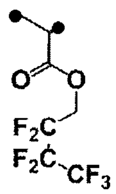 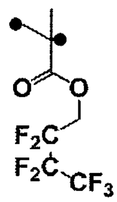 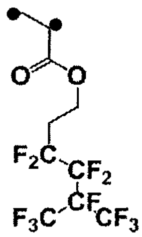 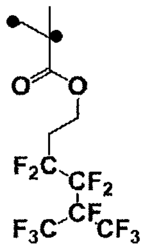

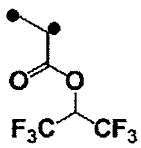 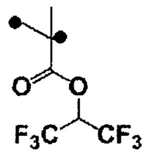

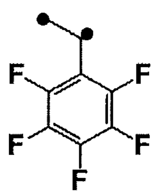 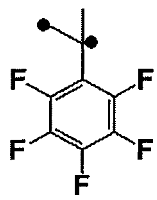 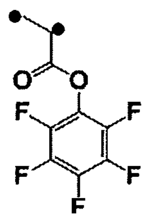 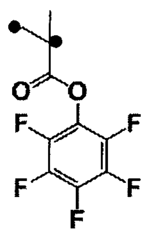

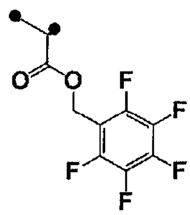 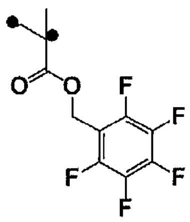 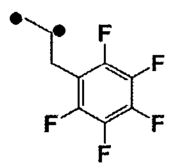

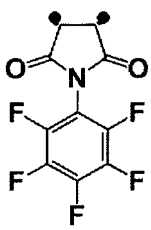 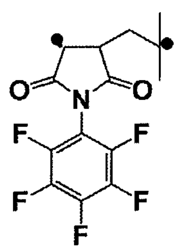 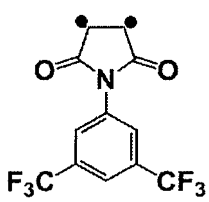 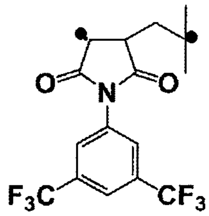

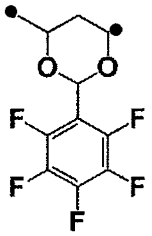 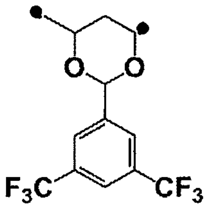 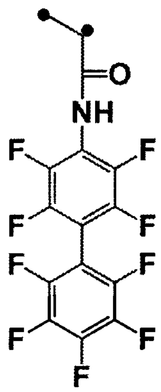 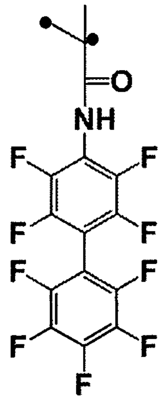

The content of the repeating unit represented by Formula (1) is preferably 10 mol% or more, more preferably 20 to 100 mol%, and still more preferably 30 to 100 mol% with respect to all the repeating units of the specific polymer from the viewpoint of further improving the sensitivity and selectivity of the sensor.

The weight-average molecular weight of the specific polymer is preferably 1,000 to 1,000,000, more preferably 5,000 to 500,000, and still more preferably 10,000 to 300,000 from the viewpoint of further improving the sensitivity and selectivity of the sensor.

The fluorine content of the specific polymer (the content of the fluorine atom of the specific polymer) is preferably 30% to 60% by mass, more preferably 35% to 60% by mass, and still more preferably 40% to 60% by mass with respect to the total mass of the specific polymer from the viewpoint of further improving the sensitivity and selectivity of the sensor.

The specific polymer can be synthesized using a known method.

[Embodiment of Sensor]

<Receiving Layer>

The sensor according to the embodiment of the present invention has a receiving layer containing a specific polymer. As long as the sensor has a predetermined receiving layer, the configuration thereof is not particularly limited, but the sensor preferably has at least a sensor main body and a receiving layer containing a specific polymer. The sensor may have members other than the predetermined receiving layer and the sensor main body.

The receiving layer containing the specific polymer can selectively adsorb a ketone-based compound. It is preferable that the sensor according to the embodiment of the present invention detects a ketone-based compound using such properties of the receiving layer containing the specific polymer.

The content of the specific polymer in the receiving layer varies depending on the embodiment of the sensor, but the content is preferably 10% to 100% by mass, more preferably 30% to 100% by mass, and still more preferably 50% to 100% by mass with respect to the total mass of the receiving layer.

A method of forming the receiving layer containing the specific polymer is not particularly limited and examples thereof include a method of applying a composition obtained by dissolving a specific polymer in a solvent (such as acetone or tetrahydrofuran) to the surface on which the receiving layer containing the specific polymer is formed and further drying the obtained coating film to form a film.

The film thickness of the receiving layer containing the specific polymer varies depending on the embodiment of the sensor, but the film thickness is preferably 10 nm to 100 $\mu$m, more preferably 50 nm to 50 $\mu$m, and still more preferably 100 nm to 10 $\mu$m.

The sensor according to the embodiment of the present invention preferably has another receiving layer, in addition to the receiving layer containing the specific polymer. In a case where the sensor has another receiving layer having different properties, for example, there are advantages such as that compounds other than a ketone-based compound can be detected and the measurement accuracy for a composite gas can be improved. The receiving layer containing the specific polymer and another receiving layer may be separately arranged or may be laminated.

The embodiment of the sensor according to the present invention is not particularly limited as long as the sensor has a receiving layer containing a specific polymer, and the sensor is preferably used as a gas sensor that detects a gas of a ketone-based compound in the air.

Specific representative examples of the embodiment of the sensor according to the present invention include a resonant sensor, an electrical resistance type sensor, a field effect transistor type sensor, and a concentration desorption type sensor.

Hereinafter, these forms will be described.

<Resonant Sensor>

The resonant sensor adsorbs a specific type of gas molecule contained in the air onto the surface, and takes the presence or absence of adsorption or the amount of adsorption as a decrease amount in the resonance frequency of the dielectric material (piezoelectric material) to be resonantly driven so as to detect the target gas. That is, the resonant sensor is a sensor using a mass micro-balancing method.

Fig. 1 is a cross-sectional view schematically showing an example of a laminate structure in a case where the sensor according to the embodiment of the present invention is a resonant sensor. The resonant sensor shown in Fig. 1 has a laminate structure in which a first electrode 1, a dielectric material 2, a second electrode 3, and a receiving layer 4 containing a specific polymer are provided in this order. In addition, a substrate for supporting the resonant sensor may be provided on a surface of the first electrode 1 opposite to the dielectric material 2. In a case where the dielectric material is of a self-oscillation type, the substrate is not required. On the other hand, in a case where the dielectric material is a ceramic piezoelectric element, or the like, the substrate is required to resonantly drive the element.

In the sensing by the mass micro-balancing method, a voltage is applied to a fine dielectric material (piezoelectric material) to vibrate the dielectric material at a constant frequency (resonance frequency), and the mass increase due to gas adsorption onto the surface of the dielectric material is detected as a decrease in the resonance frequency. As a representative example of a sensor using a mass micro-balancing method, there is known a sensor using a quartz crystal mass micro-balancing (QCM) method using a crystal as a dielectric material for resonance driving (quartz crystal microbalance sensor).

The QCM sensor is usually provided with electrodes on both sides of a crystal thin film cut at a specific angle (AT-cut), and a voltage is applied to cause shear vibration at a resonance frequency in the horizontal direction with the crystal surface. Since this resonance frequency decreases with the mass of gas adsorbed on the electrode, a change in mass of the substance on the electrode can be captured. The QCM sensor itself having a crystal vibrator including crystals and an electrode sandwiched between the crystals is known, and can be produced by a normal method or a commercially available product may be used.

A QCM sensor as one embodiment of the sensor according to the present invention preferably has a receiving layer containing a specific polymer for adsorbing a ketone-based compound onto the surface of one electrode of a pair of electrodes provided to sandwich a dielectric material therebetween. That is, as the sensor according to the embodiment of the present invention, a resonant sensor having a crystal resonator and a receiving layer arranged on the crystal resonator is preferable. The mass of the ketone-based compound, which is adsorbed onto the receiving layer containing the specific polymer, is detected as a decrease in the resonance frequency of the crystal vibrator to be resonantly driven.

The electrode used in the resonant sensor is not particularly limited and a metal material or the like usually used as an electrode can be used.

In addition to the QCM sensor as the resonant sensor, a resonant sensor formed of a ceramic dielectric (piezoelectric material) without using a crystal or quartz as the dielectric material can be employed. Examples of such sensors include a cantilever type sensor and a surface acoustic wave (SAW) sensor. Since a ceramic dielectric material can be formed on a substrate by using a sputtering method, a vacuum deposition method, or the like, there is an advantage that the ceramic dielectric material can be applied to prepare a sensor using a micro electro mechanical system (MEMS) technology. Examples of such ceramic dielectric materials include lead zirconate titanate (PZT), lead zirconate titanate doped with niobium (PZTN), zinc oxide (ZnO), and aluminum nitride (AlN).

In a cantilever type sensor, electrodes are arranged on both sides of a film formed of the above-mentioned ceramic dielectric material, and a specific voltage is applied between the electrodes to resonantly drive the ceramic dielectric material. In a case where a resonant sensor formed of a ceramic dielectric material is used as the sensor according to the embodiment of the present invention, it is preferable to arrange a receiving layer containing a specific polymer for adsorbing a ketone-based compound on the surface of one electrode of the pair of electrodes provided to sandwich the dielectric material therebetween. The mass of the ketone-based compound, which is adsorbed onto the receiving layer containing the specific polymer, is detected as a decrease in the resonance frequency of the ceramic dielectric material to be resonantly driven.

<Electrical Resistance Type Sensor>

An electrical resistance type sensor is an electrical resistance type sensor in which electrodes provided on a substrate are connected by an electrically conductive receiving layer, a voltage is applied between the electrodes, and the adsorption amount of gas onto the receiving layer (in the electrical resistance type sensor as one embodiment of the sensor according to the present invention, the adsorption amount of a ketone-based compound) is regarded as an increase in electrical resistance between the electrodes. The configuration of the electrical resistance type sensor itself is known and for example, paragraphs [0023] to [0028] of JP2002-526769A can be referred to.

In the electrical resistance type sensor, for the receiving layer that connects the two electrodes, electrical

conductivity is required. Therefore, it is preferable that the receiving layer of the electric resistance type sensor as one embodiment of the present invention contains a conductive material together with the specific polymer.

The conductive material may be an organic conductive material, an inorganic conductive material, or a mixed conductive material of an inorganic material and an organic material. Specific examples of these conductive materials include materials described in [Table 2] in paragraph [0028] of JP2002-526769A.

Moreover, as an electric resistance type sensor substrate, an insulating substrate is preferable, and for example, a glass substrate can be used.

The electrode used in the electric resistance type sensor is not particularly limited and a metal material or the like usually used as an electrode can be used.

**Field effect Transistor Type Sensor**

A field effect transistor (FET) type sensor as one embodiment of the sensor according to the present invention is preferably provided with a receiving layer containing a specific polymer in contact with a semiconductor layer of the transistor. The adsorption of a ketone-based compound onto the receiving layer containing a specific polymer can be detected as an increase in the resistance of the semiconductor layer.

Fig. 2 is a cross-sectional view schematically showing a configuration of a FET type sensor as an embodiment of the sensor according to the present invention. As shown in Fig. 2, a transistor 10 has a substrate 20, a gate electrode 22 arranged on the substrate 20, a gate insulation layer 24 arranged so to as to cover gate electrode 22, a semiconductor layer 26 arranged on the gate insulation layer 24, a source electrode 28 and a drain electrode 30 arranged to be separated from each other on the semiconductor layer 26, and a receiving layer 32 containing a specific polymer which is arranged on the source electrode 28, the drain electrode 30, and the semiconductor layer 26. The transistor 10 is a so-called bottom gate-top contact type transistor.

In the FET type sensor, in a case where a ketone-based compound is adsorbed onto the receiving layer containing a specific polymer, the electrical resistance of the semiconductor layer arranged adjacent to the receiving layer containing a specific polymer is changed and as a result, the electrical properties of the transistor are changed. By detecting the change in the electrical properties of the transistor, a ketone-based compound can be detected.

The kind of the change in the electrical properties of the transistor is not particularly limited and examples thereof include a change in current value between the source electrode and the drain electrode (current value of drain current), a change in carrier mobility, and a voltage change. Among these, from the viewpoint of easy measurement, it is preferable to detect a change in current value between the source electrode and the drain electrode (current value of drain current).

<Concentration Desorption Type Sensor>

In addition, a concentration desorption type sensor that adsorbs and concentrates a compound to be detected onto a receiving layer, then desorbs the concentrated compound from the receiving layer, and performs measurement with a sensor main body can also be included as one embodiment of the present invention.

The receiving layer used in such use is particularly referred to as a receiving concentration layer.

For example, the concentration desorption type sensor as one embodiment of the present invention adsorbs and concentrates a ketone-based compound onto a receiving concentration layer containing a specific polymer, then desorbs the concentrated ketone-based compound from the receiving concentration layer containing a specific polymer, and performs measurement with a sensor main body.

As a method of concentrating a ketone-based compound as a compound to be detected in the receiving concentration layer containing a specific polymer, for example, a method in which until a sufficient amount of ketone-based compound is adsorbed onto the receiving concentration layer containing a specific polymer, the receiving concentration layer containing a specific polymer is continuously exposed to a gas containing a ketone-based compound for a predetermined period of time may be used.

As a method of desorbing the concentrated ketone-based compound, for example, a method of heating the receiving concentration layer containing a specific polymer may be used.

As the sensor main body for measuring the desorbed ketone-based compound, the above-described various sensors may be further used and other measurement means may be used.

In this manner, an embodiment in which the receiving layer containing a specific polymer (including the receiving concentration layer containing a specific polymer) is present while being separated from the sensor main body is also included in the sensor in the present specification.

Since such a concentration desorption type sensor as one embodiment of the sensor according to the present invention has a step of concentrating a ketone-based compound, even a very small amount of ketone-based compound can be measured with higher accuracy. Such a sensor is also preferably used as, for example, a skin gas measurement

sensor for measuring a ketone-based compound in skin gas.

In the present specification, the skin gas is a general term for volatile substances released from the body surface.

(Skin Gas Measurement Sensor)

Fig. 3 shows an embodiment in a case where a concentration desorption type sensor (skin gas measurement sensor) for measuring a ketone-based compound in skin gas is adopted as the sensor according to the present invention.

In the embodiment, the sensor according to the present invention includes a sensor main body 100, a skin gas collecting unit 101, a skin gas concentrating unit 102, a receiving concentration layer containing a specific polymer 103, a heating unit 104, and an opening 106.

First, a ketone-based compound in a skin gas released from a skin surface 105 is collected from the opening 106 closely attached to the skin surface to the skin gas collecting unit 101 for a predetermined period of time. The ketone-based compound in the collected skin gas is adsorbed and concentrated by the receiving concentration layer containing a specific polymer 103 of the skin gas concentrating unit 102 present in the skin gas collecting unit 101. Then, the receiving concentration layer containing a specific polymer 103 onto which the ketone-based compound in the skin gas is adsorbed is heated by the heating unit 104 to desorb the ketone-based compound. Next, the desorbed ketone-based compound is introduced into the sensor main body 100 and the amount of the ketone-based compound in the skin gas is measured.

[Use]

The use of the sensor according to the embodiment of the present invention is not particularly limited and for example, the sensor may be used for exhalation or skin gas inspection, odor quantitative measurement, gas leak inspection, environmental investigation, and the like.

Among these, the sensor according to the embodiment of the present invention is preferably used as a sensor for detecting a ketone-based compound included in exhalation or skin gas by using the excellent sensitivity and selectivity of the sensor according to the embodiment of the present invention with respect to a ketone-based compound.

[Ketone-Based Compound]

The kind of the ketone-based compound detected by the sensor according to the embodiment of the present invention is not particularly limited, and examples thereof include acetone, methyl ethyl ketone, methyl isopropyl ketone, methyl isobutyl ketone, methyl n-butyl ketone, methyl amyl ketone, cyclopentanone, cyclohexanone, and isophorone.

Among these, the sensor according to the embodiment of the present invention is preferably used for detection of acetone.

It is known that there is a possibility that acetone may be high in, for example, the exhalation and skin gas of diabetics, and such a method of measuring acetone in exhalation or skin gas is expected as a safe and simple diagnostic method.

Examples

Hereinafter, the present invention will be described in more detail based on Examples. The materials, amounts used, ratios, processing details, processing procedures, and the like shown in the following Examples can be changed as appropriate without departing from the spirit of the present invention. Accordingly, the scope of the present invention should not be construed as being limited by the examples shown below.

[Example 1]

<Test>

2,3,4,5,6-pentafluorostyrene (manufactured by Tokyo Chemical Industry Co., Ltd.) was polymerized in methyl ethyl ketone (MEK, manufactured by Wako Pure Chemical Industries, Ltd.) using V-601 (manufactured by Wako Pure Chemical Industries, Ltd.) as an initiator to obtain a polymer P-01 (weight-average molecular weight: 50,000).

A mixed solution was obtained by dissolving the polymer P-01 (20 mg) in acetone (40 g). The obtained mixed solution was added dropwise onto one surface of a crystal vibrator in a QCM sensor (manufactured by Tamadevice. co. ltd.) and further dried at room temperature to form a film formed of the polymer P-01 as a receiving layer. The QCM sensor having the receiving layer obtained was put into a flow cell, 100 vol ppm of various test gases shown below were allowed to pass using nitrogen as a carrier, and the sensitivity and selectivity of the QCM sensor having the receiving

layer with respect to various test gases were evaluated. The results are shown in Table 1.

<Test Gas>

The test gases used for evaluation are as follows.
Acetone
Hexane
Toluene
Ethanol

<Evaluation>

(Sensitivity)

In a case where acetone was formed of as a test gas, based on the absolute value of the frequency change occurred in the QCM sensor having a receiving layer, the sensitivity was evaluated according to the standards shown below. Here, as the absolute value of the frequency change increases, the sensitivity of the sensor becomes further excellent.

5: 50 Hz or more
4: 30 Hz or more and less than 50 Hz
3: 20 Hz or more and less than 30 Hz
2: 10 Hz or more and less than 20 Hz
1: Less than 10 Hz

(Selectivity)

• Selectivity 1 (to Hexane)

Based on the ratio between the absolute value of the frequency change in a case of using acetone as a test gas and the absolute value of the frequency change in a case of using hexane as a test gas (= the absolute value of frequency change in a case of using acetone/the absolute value of frequency change in a case of using hexane), the selectivity was evaluated according to the following standards. As the ratio value increases, the selectivity of the sensor becomes further excellent.

5: 20 or more
4: 13 or more and less than 20
3: 6 or more and less than 13
2: 2 or more and less than 6
1: Less than 2

. Selectivity 2 (to Toluene)

Based on the ratio between the absolute value of the frequency change in a case of using acetone as a test gas and the absolute value of the frequency change in a case of using toluene as a test gas, the selectivity was evaluated according to the same standards as shown in Selectivity 1.

• Selectivity 3 (to Ethanol)

Based on the ratio between the absolute value of the frequency change in a case of using acetone as a test gas and the absolute value of the frequency change in a case of using ethanol as a test gas, the selectivity was evaluated according to the same standards as shown in Selectivity 1.

[Examples 2 to 7]

The following polymers P-02 to P-07 were synthesized using a known method as in Example 1.
The weight-average molecular weight of each polymer was as follows.
P-02: 40,000, P-03: 30,000, P-04: 50,000, P-05: 80,000, P-06: 70,000, P-07: 60,000.

QCM sensors having a receiving layer were prepared and evaluated in the same manner as in Example 1 using each polymer obtained. The results are shown in Table 1.

[Comparative Examples 1 to 3]

QCM sensors having a receiving layer were prepared and evaluated in the same manner as in Example 1 except that polymers D-01 to D-03 shown below were used as polymers. The results are shown in Table 1.

As the polymer D-01 (weight-average molecular weight: 20,000), a commercially available product (manufactured by Wako Pure Chemical Industries, Ltd.) was used.

The polymer D-02 (weight-average molecular weight: 100,000) was synthesized using a known method.

As the polymer D-03, a commercially available product (Teflon AF2400, manufactured by DuPont) was used.

Hereinafter, the structures of the polymers P-01 to P-07 and the polymers D-01 to D-03 are shown below.

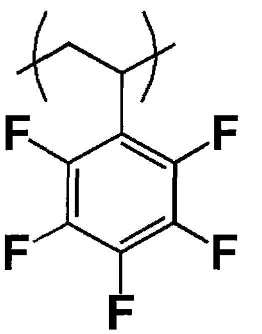

P-01

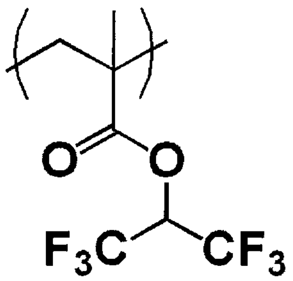

P-02

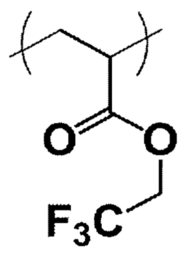

P-03

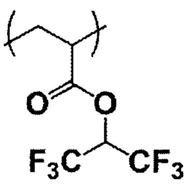

P-04

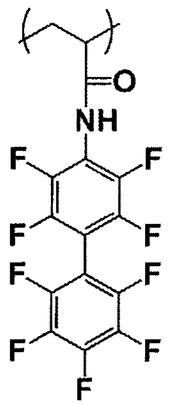

P-05

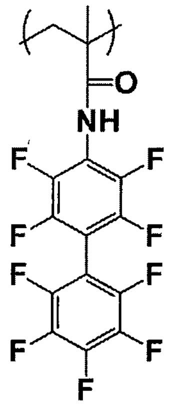

P-06

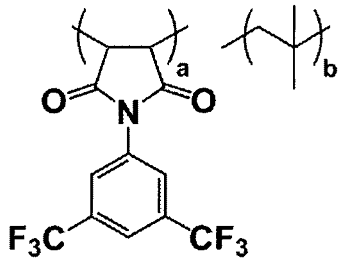

a/b=0.5/0.5

P-07

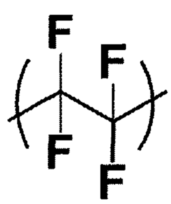

D-01

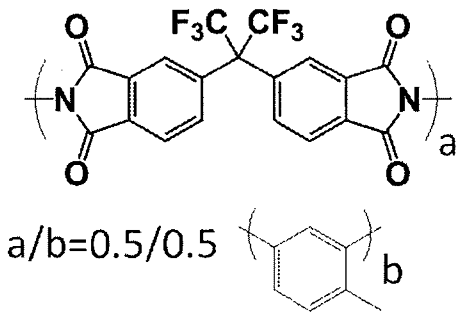

a/b=0.5/0.5

D-02

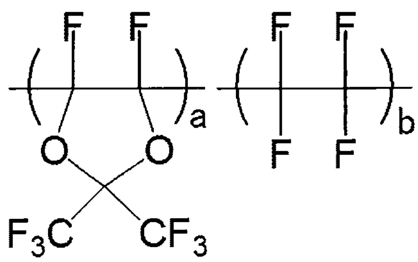

a/b=0.87/0.13

D-03

The evaluation results of the sensors prepared in each of Examples and Comparative Examples shown are shown in Table 1 below.

The fluorine content in the repeating unit was calculated according to the following expression.

$$\text{Fluorine content (\% by mass)} = 100 \times \text{(total mass of fluorine atom of repeating unit having fluorine atom/total mass of repeating unit having fluorine atom)}$$

However, in the polymers P-07 and D-2, the repeating unit having no fluorine atom is not included in the calculation of the fluorine content. In the polymer D-3, the fluorine content is described for two types of repeating units having a fluorine atom.

[Table 1]

| | Polymer | Fluorine content in repeating unit (% by mass) | Sensitivity | Selectivity 1 (to Hexane) | Selectivity 2 (to Toluene) | Selectivity 3 (to Ethanol) |
|---|---|---|---|---|---|---|
| Example 1 | P-01 | 49 | 5 | 5 | 5 | 5 |
| Example 2 | P-02 | 48 | 5 | 5 | 5 | 5 |
| Example 3 | P-03 | 34 | 3 | 3 | 3 | 3 |
| Example 4 | P-04 | 51 | 5 | 5 | 5 | 5 |
| Example 5 | P-05 | 44 | 5 | 5 | 5 | 5 |
| Example 6 | P-06 | 43 | 5 | 5 | 5 | 5 |
| Example 7 | P-07 | 37 | 4 | 4 | 4 | 3 |
| Comparative Example 1 | D-01 | 76 | 1 | 1 | 1 | 1 |
| Comparative Example 2 | D-02 | 26 | 2 | 1 | 1 | 1 |
| Comparative Example 3 | D-03 | 62/76 | 1 | 1 | 1 | 1 |

As shown in Table 1, it was confirmed that the sensor according to the embodiment of the present invention exhibited excellent sensitivity and selectivity with respect to a ketone-based compound.

In addition, it was confirmed that in a case where the fluorine content of the repeating unit represented by Formula (1) was 40% by mass or more, the sensitivity and selectivity of the sensor with respect to a ketone-based compound were further excellent.

Explanation of References

1: first electrode

2: dielectric material (piezoelectric material)

3: second electrode

4: receiving layer containing specific polymer

10: transistor

20: substrate

22: gate electrode

24: gate insulation layer

26: semiconductor layer

28: source electrode

30: drain electrode

32: receiving layer containing specific polymer

100: sensor main body

101: skin gas collecting unit

102: skin gas concentrating unit

103: receiving concentration layer containing specific polymer

104: heating unit

105: skin surface

106: opening

**Claims**

1. A sensor that detects a ketone-based compound, comprising:

a receiving layer that contains a polymer having a repeating unit represented by Formula (1),
wherein a fluorine content of the repeating unit represented by Formula (1) shown later is 30% to 60% by mass,

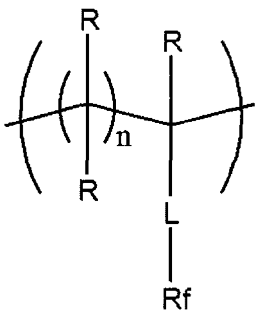

(1)

n represents an integer of 1 to 3,
R's each independently represent a hydrogen atom or an organic group, R's may be linked to each other to form a ring,
L represents a single bond or a divalent linking group, L may be linked to any one of R's to form a ring,
where neither R nor L has a fluorine atom, and
Rf represents an organic group having three or more fluorine atoms.

2. The sensor according to claim 1,
wherein a content of the repeating unit represented by Formula (1) is 10 mol% or more with respect to all the repeating units of the polymer.

3. The sensor according to claim 1 or 2,
wherein the fluorine content of the repeating unit represented by Formula (1) is 40% to 60% by mass.

4. The sensor according to any one of claims 1 to 3,
wherein Rf represents a fluorohydrocarbon group having three or more fluorine atoms.

5. The sensor according to any one of claims 1 to 4, wherein Rf represents a fluorohydrocarbon group that has three or more fluorine atoms and is selected from the group consisting of an aryl group, an alkyl group, and a group formed by combining a plurality thereof.

6. The sensor according to any one of claims 1 to 5, wherein Rf represents a fluorinated aryl group having three or more fluorine atoms, a fluorinated alkyl group having three or more fluorine atoms, a fluorinated biphenyl group having three or more fluorine atoms, a fluorinated alkyl-substituted aryl group having three or more fluorine atoms, or a fluorinated benzyl group having three or more fluorine atoms.

7. The sensor according to any one of claims 1 to 6, wherein Rf represents a fluorinated aryl group having three or more fluorine atoms.

8. The sensor according to any one of claims 1 to 7, wherein Rf represents a pentafluorophenyl group.

9. The sensor according to any one of claims 1 to 8, wherein L represents a single bond, an ester group, an amide group, or a group that is linked to any one of R's to form a ring.

10. The sensor according to any one of claims 1 to 9, wherein L represents a single bond, an ester group, or an amide group.

11. The sensor according to any one of claims 1 to 10 that detects a ketone-based compound contained in exhalation or skin gas.

12. The sensor according to any one of claims 1 to 11, wherein the ketone-based compound is acetone.

13. The sensor according to any one of claims 1 to 12, further comprising: another receiving layer, in addition to the receiving layer.

14. The sensor according to any one of claims 1 to 13 that is a resonant sensor.

FIG. 1

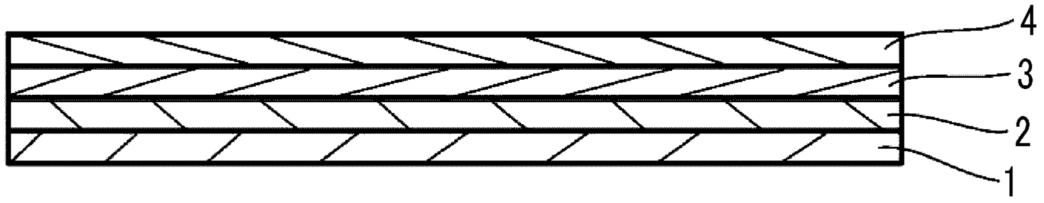


FIG. 2

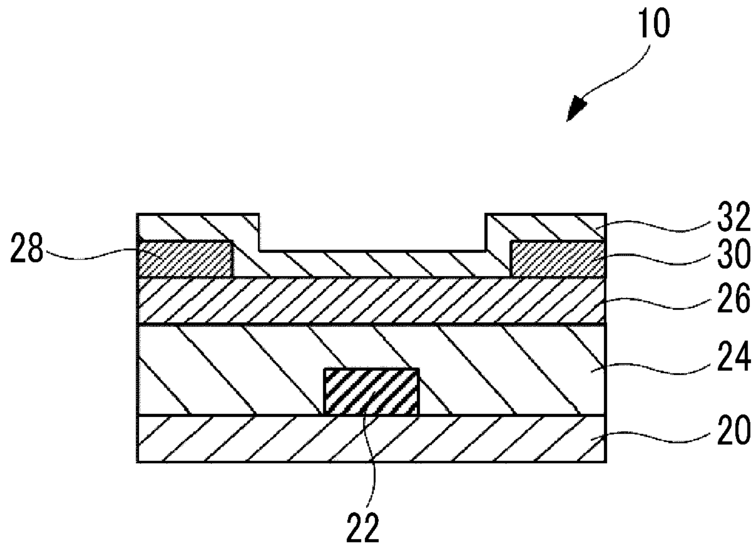


FIG. 3

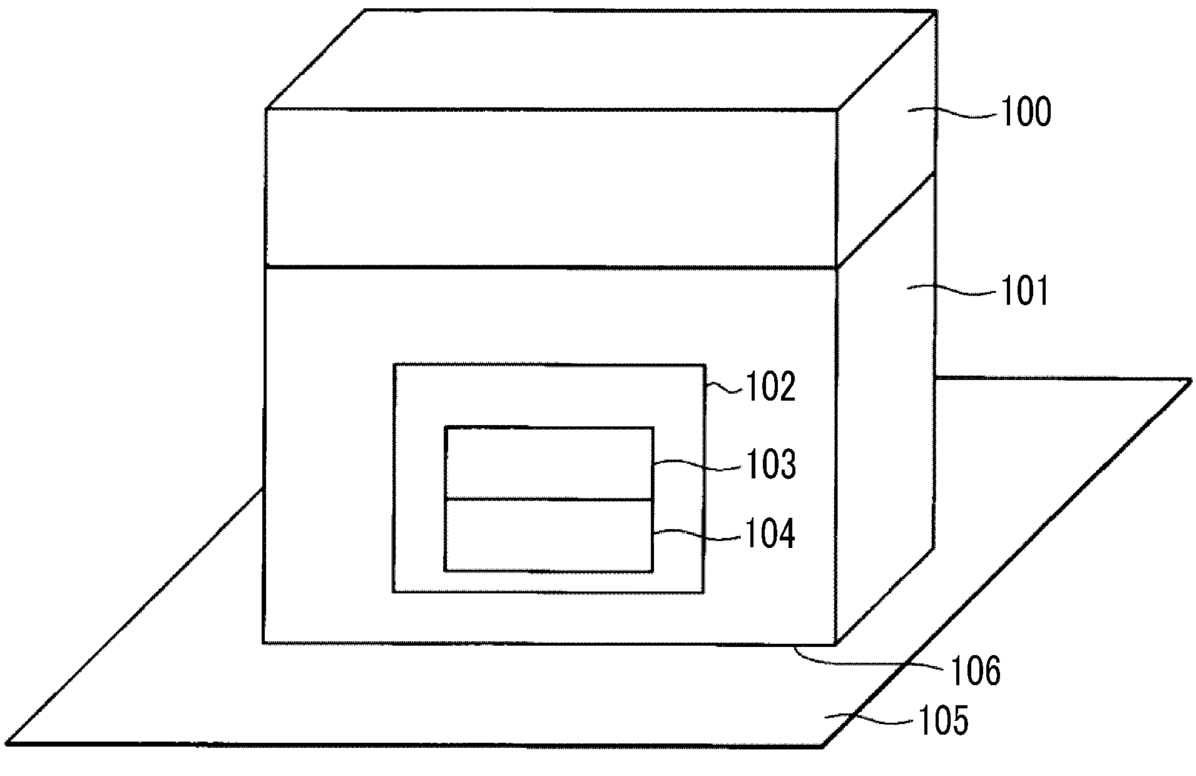

**INTERNATIONAL SEARCH REPORT**

International application No.
PCT/JP2018/027866

A. CLASSIFICATION OF SUBJECT MATTER
Int.Cl. G01N5/02(2006.01)i, G01N27/22(2006.01)i

According to International Patent Classification (IPC) or to both national classification and IPC

B. FIELDS SEARCHED

Minimum documentation searched (classification system followed by classification symbols)
Int.Cl. G01N5/02, G01N27/22

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | SEVERIN, E. J. et al., "An Investigation of the Concentration Dependence and Response to Analyte Mixtures of Carbon Black/Insulating Organic Polymer Composite Vapor Detectors", Analytical Chemistry,, 2000, vol. 72, pp. 658-668 | 1-10, 13<br>11-12, 14 |
| X<br>Y<br>A | NANTO, H. et al., "Plasma-polymerized-film Coated QCM Gas Sensor for Environmental Monitoring", Chemical Sensors, 2001, vol. 17, supplement B, pp. 363-365, 1PB13 | 1-6, 9-10, 12, 14<br>11, 13<br>7-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 October 2018 (16.10.2018) | 30 October 2018 (30.10.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/027866

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | JP 8-277311 A (HOECHST IND KK) 22 October 1996, claim 1, paragraphs [0011], [018]-[0026] & EP 736768 A2, claim 1, page3, lines 9-11, examples 1-2 | 1-6, 9-10, 12<br>11, 13<br>14 |
| Y | WO 2008/126519 A1 (SHINSHU UNIVERSITY) 23 October 2008, paragraph [0002] & US 2010/0024533 A1, paragraph [0002] | 11 |
| Y | DNESHKAH, A. et al., "Poly(vinylidene fluoride-1exafluoropropylene) composite sensors forvolatile organic compounds detection in breath", Sensors and Actuators B:, 2015, vol. 221, pp. 635-643 | 11 |
| A | GRAUNKE, T. et al., "Towards Enhanced Gas Sensor Performance with Fluoropolymer Membranes", Sensors, 2016, vol. 16, 1605, pp. 1-20 | 1-14 |
| A | LONERGAN, M. C. et al., "Array-Based Vapor Sensing Using :hemically Sensitive, Carbon Black-Polymer Resistors", Chem. Vlater, 1996, vol. 8, pp. 2298-2312 | 1-14 |
| A | JP 2014-190914 A (SEIKO EPSON CORP.) 06 October 2014, claims (Family: none) | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015007618 A
- JP 2002526769 A